# EUROPÄISCHE PATENTSCHRIFT

(11) **EP 2 416 707 B1**
(45) Veröffentlichungstag und Bekanntmachung des Hinweises auf die Patenterteilung: **05.10.2016**
(21) Anmeldenummer: 10711195.7
(22) Anmeldetag: 19.03.2010
(51) Int. Cl.: A61B 5/08, G01N 33/00, A61B 5/097, G01N 27/414, G01N 33/497

(54) **GAS-ANALYSEGERÄT MIT EINER KOMBINATION AUS GASENTFEUCHTER UND GASKONVERTER**
GAS ANALYSIS APPARATUS HAVING A COMBINATION OF GAS DEHUMIDIFIER AND GAS CONVERTER
APPAREIL D'ANALYSE DE GAZ COMBINANT UN DÉSHUMIDIFICATEUR DE GAZ ET UN CONVERTISSEUR DE GAZ

(30) Priorität: 08.04.2009 DE 102009016848
(43) Veröffentlichungstag der Anmeldung: 15.02.2012
(73) Patentinhaber: Robert Bosch GmbH, 70442 Stuttgart (DE)
(72) Erfinder: ABRAHAM-FUCHS, Klaus, 91058 Erlangen (DE); FLEISCHER, Maximilian, 85635 Höhenkirchen (DE); HILTAWSKY, Karsten, 58239 Schwerte (DE); HORNUNG, Oliver, 90768 Fürth (DE); KRÜGER-SUNDHAUS, Thomas, 96178 Pommersfelden (DE); MAGORI, Erhard, 85622 Feldkirchen (DE); PAULICKA, Peter, 91341 Röttenbach (DE); VON SICARD, Oliver, 80469 München (DE)
(86) Internationale Anmeldenummer: PCT/EP2010/053590
(87) Internationale Veröffentlichungsnummer: WO 2010/115694

(56) Entgegenhaltungen:
- WO-A1-97/35519
- WO-A1-99/57560
- WO-A1-2008/088780
- WO-A2-2006/114766
- DE-A1- 10 130 296
- DE-U1- 9 320 785
- US-A1- 2008 093 226
- OLEKSANDR KUZMYCH ET AL: "Carbon nanotube sensors for exhaled breath components" NANOTECHNOLOGY, IOP, BRISTOL, GB LNKD- DOI:10.1088/0957-4484/18/37/375502, Bd. 18, Nr. 37, 19. September 2007 (2007-09-19), Seite 375502, XP020119566 ISSN: 0957-4484

## Beschreibung

Aus WO 2008/088780 A1, OLEKSANDR KUCMYCH et al. "Carbon Nanotube Sensors for exhaled breath components", Nanotechnology 18 (2007) 375502, WO 97/35519 und DE 10130296 A1 sind Vorrichtungen zur Bestimmung von Stickstoffmonoxid in Ausatemluft bekannt.

Aus WO 99/57560 ist eine Vorrichtung zum Transport von Gasproben bekannt.

Aus WO 2006/114766 A2 ist eine Vorrichtung zur Bestimmung von Stickstoffmonoxid über eine Konvertierung des Stickstoffmonoxids in Stickstoffdioxid bekannt.

US 2008/0093226 A1 offenbart Nanoelektronische Sensoren zur Detektion der Analyten beispielsweise Ammoniak.

Aus DE 9320785 U1 ist ein Gerät zur kontrollierten Zudosierung von Stickstoffmonoxid zur Atemluft von Patienten bekannt.

Die vorliegende Erfindung betrifft eine Anordnung zur Messung der Konzentration von Stickstoffmonoxid (NO) im Atemgas und ein Verfahren zur Messung der Konzentration von NO.

Stickoxid (Stickstoffmonoxid, NO) wird in sehr geringen Mengen aus den Zellen der Atemwege kontinuierlich in den Atemgasstrom freigesetzt. Für die Diagnose und optimierte Therapie von Asthma und anderen inflammatorischen Atemwegserkrankungen stellt NO einen wichtigen Marker dar. Asthma gehört bei ca. 5% der Erwachsenen und ca. 20% der Kinder in entwickelten Industrienationen zu den am häufigsten auftretenden Krankheiten. Bei Entzündungsvorgängen der Atemwege, z.B. Asthma treten erhöhte NO-Konzentrationen von 80 ppb (parts per billion) in der Ausatemluft auf. Bevorstehende Asthmaanfälle sind durch einen Anstieg des NO-Gehaltes der Ausatemluft bereits deutlich früher erkennbar als durch einen Lungenfunktionstest. Somit ist die NO-Messung in der Ausatemluft ein bevorzugtes Verfahren zur Diagnose und Therapieverlaufskontrolle von Asthma und anderen entzündlichen Atemwegserkrankungen.

Preiswerte NO-Sensoren mit der erforderlichen Empfindlichkeit im ppb-Bereich waren bisher nicht am Markt verfügbar. Ein neu entwickelter NO₂-Sensor auf Basis der "Suspended Gate FET"-Technologie entspricht den genannten Anforderungen. Allerdings muss einem derartigen Sensor ein Konversionsmodul zur Umwandlung des NO im Atemgas zu NO₂, welches vom Sensor detektiert werden kann, vorgeschaltet werden. Ein derartiges Konversionsmodul sollte idealerweise mehrere Monate oder gar Jahre halten, preiswert sein und NO mit höchstmöglicher und konstanter Umwandlungsrate in NO₂ konvertieren.

Die Umsetzung von Stickstoffmonoxid zu Stickstoffdioxid erfolgt gemäß der folgenden Reaktionsgleichung:

2 NO + O₂ ↔ 2 NO₂,

Die Umsetzung von Stickstoffmonoxid zu Stickstoffdioxid kann in einem Atemgassensorgerät durch eine Einrichtung zur Oxidation von Stickstoffmonoxid zu Stickstoffdioxid erfolgen, z.B. durch Durchleiten der (Atem-)-Luft durch ein Oxidationsmittel (z.B. Kaliumpermanganat) oder einen Oxidationskatalysator.

Ein weiteres Problem ist die Tatsache, dass sich NO₂ wesentlich besser in Wasser löst als NO. Daher wird ein Verfahren benötigt, um die Konzentration des konvertierten NO₂ im feuchten Atemgas möglichst konstant und quantitativ messbar zu halten. Aufgrund der höheren Löslichkeit von NO₂ in Wasser wird in (Atem-) Luft mit hohem Feuchtigkeitsgehalt ein Teil des (konvertierten) NO₂ in Wasser gelöst, die Konzentration des messbaren NO₂ sinkt und es wird ein scheinbar zu niedriger NO Gehalt gemessen.

Um eine quantitative Messung des NO-Gehaltes zu ermöglichen wird erfindungsgemäß ein Atemgas - Analysegerät vorgeschlagen, bei dem die Atemluft zuerst durch eine Einrichtung zur Luftentfeuchtung und anschließend durch eine Einrichtung zur Oxidation von Stickstoffmonoxid zu Stickstoffdioxid geleitet wird.

Neben der Stabilisierung und Verringerung der Menge des NO₂, das sich in der Luftfeuchtigkeit löst, hat dieses Verfahren den weiteren Vorteil, dass durch die Entfeuchtung der Luft eine eventuell vorhandene Querempfindlichkeit des Gassensors auf Luftfeuchte vermindert wird, bzw. durch die definierte Einstellung der Luftfeuchte auf einen definierten Wert gebracht wird. Zum anderen werden dadurch optional Geräte möglich, deren Energiebedarf wesentlich reduziert ist: Atem mit einem Taupunkt von typischerweise 35-38°C würde in einem sich auf Raumtemperatur befindlichen Gerat eine Kondensation der

Feuchte bewirken, die gegebenenfalls. zu Meßverfälschungen oder aber wenn die Meßkammer gut gegenüber der Umgebung abgeschlossen ist zu einem Voll-Laufen der Meßkammer mit Wasser führen. Üblicherweise wird zur Verhinderung dieses Effektes eine Heizung der Kammer über die Taupunkttemperatur der Atemfeuchte eingesetzt, die einen Energiebedarf von typischerweise mehreren W mit sich bringt, sowie zu einer Wartezeit bis zur Betriebsbereitschaft führt (Aufheizphase). Durch den Gasentfeuchter kann darauf verzichtet werden und ein kleines Gerat mit vermindertem Energiebedarf konstruiert werden.

Die Erfindung betrifft insbesondere eine Vorrichtung zur Messung von Stickstoffmonoxid in einer Ausatemluft durch eine Gassensoreinheit mit mindestens einem Gassensor, aufweisend eine Einrichtung zur Oxidation von Stickstoffmonoxid zu Stickstoffdioxid, wobei der Einrichtung zur Oxidation von Stickstoffmonoxid zu Stickstoffdioxid eine Einrichtung zur Luftentfeuchtung vorgeschaltet ist.

Ganz allgemein betrifft die Erfindung eine Vorrichtung zur Messung eines Gasanalyten in Ausatemluft durch eine Gassensoreinheit mit mindestens einem Gassensor, aufweisend eine Einrichtung zur Gaskonversion, wobei der Einrichtung zur Gaskonversion eine Einrichtung zur Luftentfeuchtung vorgeschaltet ist.

Erfindungsgemäß ist der Gasanalyt Stickstoffmonoxid und die Einrichtung zur Gaskonversion eine Einrichtung zur Oxidation von Stickstoffmonoxid zu Stickstoffdioxid.

Bevorzugt ist der Gassensor ein N02-sensitiver FET-Sensor.

Die Oxidation kann durch Durchleiten der (Atem-)-Luft durch ein Oxidationsmittel (z.B. Kaliumpermanganat) oder einen Oxidationskatalysator erfolgen.

Bevorzugt ist ein Indikator vorgesehen, der den Grad der Wasseraufnahme anzeigt oder anzeigt, wenn der Wassergehalt der
Luft nach Durchströmen der Einrichtung zur Luftentfeuchtung einen kritischen Schwellenwert übersteigt.

Gemäß einer Ausführungsform wird ein chemisches Trocknungsmittel zur Luftentfeuchtung verwendet.

Es gibt verschiedene Typen von Trocknungsmitteln:
- Trocknung über eine chemische Reaktion = irreversibel
- Wasser wird über Kristallwasserbildung gebunden
- reversibel in Molekularsieben absorbiert

Geeignete Trocknungsmittel sind insbesondere Calciumchlorid, Kupfersulfat, Silica oder Zeolithe, Blaugel, Orangegel und ähnliche. Blaugel und Orangegel bestehen ebenfalls aus Silicagel, enthalten jedoch einen Indikator-Farbstoff, der den Grad der Wasseraufnahme anzeigt.

Gemäß einer Ausführungsform der Erfindung ist die Einrichtung zur Luftentfeuchtung als Verbrauchsmittel in einer separaten Einheit vorgesehen, welche bei Verbrauch der Einrichtung, z.B. bei Erschöpfung der Wasseraufnahmekapazität, separat ausgetauscht werden kann.

Gemäß einer Ausführungsform der Erfindung ist die Einrichtung zur Luftentfeuchtung ein elektrisches Trocknungsmittel zur Luftentfeuchtung, z.B. ein elektrisches Heiz- oder Kondensationsmittel.

Gemäß einer Ausführungsform der Erfindung ist die Einrichtung zur Oxidation von Stickstoffmonoxid zu Stickstoffdioxid als Verbrauchsmittel in einer separaten Einheit vorgesehen, welche bei Verbrauch der Einrichtung, z.B. bei Erschöpfung der Oxidationskapazität, separat ausgetauscht werden kann.

Gemäß einer Ausführungsform der Erfindung ist die Einrichtung zur Luftentfeuchtung gemeinsam mit der Einrichtung zur Oxidation von Stickstoffmonoxid zu Stickstoffdioxid als Verbrauchsmittel in einer separaten Einheit vorgesehen.

Eine wie vorstehend beschriebene separate Einheit ist bevorzugt ohne weiteres Werkzeug, z.B. durch eine oder mehrere einfache Steckverbindung(en), austauschbar an dem Atemanalysegerät vorgesehen.

Gemäß einer Ausführungsform der Erfindung weist die Gassensoreinheit einen NO₂-sensitiven Feldeffektortransistor-Sensor (FET-Sensor) auf.

Da die Reaktionsenthalpie negativ ist, erfolgt die Reaktion in Richtung der Konversion zu NO₂, sie muss also nur durch einen Katalysator ermöglich werden. Dazu wird angemerkt, dass auch in ausgeatmeter Luft noch der Großteil des in der Umgebungsluft vorhandenen Sauerstoffs präsent ist, da nur ein kleiner Teil davon beim Atmen verbraucht wird.

Die Erfindung wird im Folgenden anhand von Beispielen und im Zusammenhang mit der Figur beschrieben, welche zeigt:

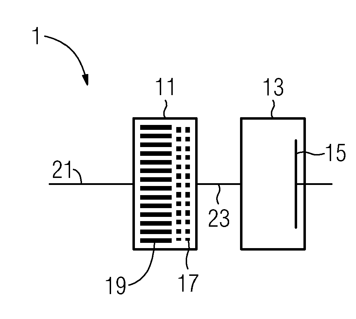

- FIG 1: eine schematische Darstellung einer Ausführungsform der erfindungsgemäßen Vorrichtung.

FIG 1 zeigt beispielhaft und schematisch eine Ausführungsform der erfindungsgemäßen Vorrichtung 1 mit einem Konversionsmodul 11 und einer Gassensoreinheit 13. Über eine Zuleitung 21 wird Ausatemluft in eine separate Einheit 11 geleitet, in welchem eine Einrichtung zur Oxidation von Stickstoffmonoxid 17 vorgesehen ist. Dieser vorgeschaltet ist eine Einrichtung zur Luftentfeuchtung 19, z.B. in Form eines chemischen Trockenmittels, wie z.B. Silica-Gel, Kupfersulfat oder ähnliches. Über eine Leitung 23 wird nach der Konversion die Ausatemluft in die Gassensoreinheit 13 geleitet, in welcher ein NO₂-sensitiver Gassensor 15 vorgesehen ist.

Optional kann die separate Einheit 11, die als Einmalartikel(Disposable) ausgeführt sein kann, oder das Flusskanalsystem, welches an die separate Einheit angesteckt wird, ein
Einwegventil (z.B. Rückschlag-Einwegventil) (nicht gezeigt) enthalten, so dass der Benutzer einmal in das Modul ausgeatmete Luft nicht mehr ansaugen und wieder einatmen kann.

Das Konverter- und oder das Entfeuchtungsmodul 11 kann so ausgestaltet sein, dass z.B. ein Farbumschlag oder eine andere wahrnehmbare Veränderung anzeigt, wann das Modul aufgebraucht ist und ausgetauscht werden muss. Eine Vergleichs- Farbskala kann neben dem Fenster zur Beobachtung des Farbumschlags angebracht sein.

Des Weiteren kann das Modul optional eine Vorrichtung zur Regenerierung der sich verbrauchenden Reaktions-Chemikalien enthalten. So kann das Entfeuchtungsmodul 11 eine Heizeinrichtung z.B. einen Heizdraht enthalten, mit dem die eingelagerte Feuchtigkeit wieder ausgeheizt werden kann. In diesem Fall sind am Modul 11 und am Analysegerät Kontakt-Schnittstellen zum Betreiben der Regenerationsvorrichtung integriert.

In einer anderen Ausführungsform wird das Konversionsmodul und/oder das Entfeuchtungsmodul 11 durch einen von außen zugänglichen Schacht direkt vor oder in die Messkammer eingeschoben, so dass das konvertierte Gas zeitnah zur Konversion gemessen werden kann und Konzentrationsänderung durch das Einstellen eine chemischen Gleichgewichts vermieden werden. In dieser Ausführungsform befindet sich das Konversionsmodul und/oder das Entfeuchtungsmodul 11 vorteilhaft hinter einem Ventil (nicht gezeigt) im Flusskanalsystem, so dass das Konversionsmodul bei geschlossenem Ventil (z.B. im passiven, unbenutzten Zustand des Messgerätes) keinen Umwelteinflüssen ausgesetzt ist.

Generell wird der Atem in der ersten Stufe durch den Entfeuchter geführt. Dieser ist so aufgebaut, dass er einen definierten und begrenzten Flußwiderstand für den Atem darstellt, sowie eine hohe Kontaktoberfläche des Trocknungsmittels zur Atemluft sicherstellt. Geeignete Geometrien stellen hier z.B. die Verwendung einer Kammer mit einer losen und offenporigen Schichtung eines Granulats dar, welches das Trocknungsmittel enthält oder aber die Verwendung einer Struktur mit einer Vielzahl von Kanälen (ähnlich den Abgaskatalysatoren), wobei die Oberfläche der Kanäle mit dem Trocknungsmittel versehen sind.

Geeignete Trocknungsmittel sind Substanzen, die stark und definiert Luftfeuchte binden, das Zielgas aber unverändert passieren lassen, z.B. Stoffe welche eine geeignete innere, Wasser bindende Porenstruktur aufweisen wie z.B. Silicagel (= Kieselgel), Zeolithe Wasser anziehende Salze und Mineralien wie z.B. Calziumchlorid, Bentonit, Tone, Wasserbindende Polymere wie z.B. getrocknete Polydextrose, Polysiloxane, wasserbindende Oxide, wie z.B. P205, S03.

Als Gassensor 15 kommt z.B. die Verwendung eines N02-sensitiven Sensors auf Basis eines Transistors in Betracht. Bei Einsatz der Stickoxiddetektion nach dem Prinzip der Austrittsarbeitsmessung sind verschiedene Feldeffekttransistoren bekannt, bei denen die gassensitive Schicht als Gate-Elektrode dargestellt ist. Diese Gate-Elektrode kann durch einen Luftspalt abgetrennt sein von dem sogenannten Kanalbereich des Feldeffekttransistors. Grundlage für ein detektierendes Messsignal ist die Änderung des Potentials zwischen Gate und Kanalbereich (ΔV_{G}). In den deutschen Patentanmeldungen Nr. 198 14 857.7 und Nr. 199 56 806.5 werden beispielsweise hybride Flip-Chip-Aufbauten von Gassensoren beschrieben, die als CMOS-Transistoren ausgeführt sind. Ein Gassensor kann darüber hinaus mit zwei Feldeffekttransistoren bestückt sein, deren Regelverhalten durch annähernd gleichgroße Luftspalte zwischen Kanalbereich und Gateelektrode angeglichen ist und deren Sensorschichten separat auslesbar sind. In der deutschen Patentanmeldung Nr. 199 56 744.1 wird beschrieben wie die Beabstandung zwischen Gate-Elektrode und Kanalbereich eines Feldeffekttransistors durch äußerst präzise Abstandshalter reproduzierbar darstellbar ist. Eine andere Ausgestaltung sieht vor, das gassensitive Material in poröser Form auf dem Kanalbereich oder dem Gate aufzubringen.

Gassensitive Schichten zum Einsatz in einem sogenannten SG-FET (Suspended Gate-Feldeffekttransistor) können vorteilhaft Porphinfarbstoffe sein, wie z.B. Phthalocyanine mit dem Zentralatom Kupfer oder Blei. Bei Sensortemperaturen zwischen 50° und 120° C können Stickoxidsensitivitäten bis in den unteren ppb-Bereich nachgewiesen werden. Die Detektion zielt wie üblich auf Stickstoffdioxid ab.

Andere zum Einsatz in gassensitiven Feldeffekttransistoren geeignete Materialien als gassensitive Schichten zur Detektion von Stickoxid, insbesondere von Stickstoffdioxid, sind bei Temperaturen zwischen 80° und 150° C betriebene feinkristalline Metalloxide. Dies können insbesondere SnO₂, WO₃, In₂O₃ sein, Salze aus dem Karbonatsystem wie Bariumkarbonat oder Polymere wie beispielsweise Polysiloxane, sind ebenfalls denkbar.

Vorzugsweise wird das Konversionsmodul möglichst nahe am Sensor, z.B. an der Einlassöffnung zur Messkammer oder in der Messkammer selbst integriert vorgesehen, damit das konvertierte Gas möglichst unmittelbar gemessen werden kann.

In einer Ausführungsform der Erfindung werden Entfeuchtungsmodul und Konvertermodul gemeinsam in einem Disposable untergebracht, derart, dass das Entfeuchtungsmodul zuerst vom Messgas durchströmt wird.

In einer alternativen Ausführungsform der Erfindung ist das Disposable modular aufgebaut, sodass das Entfeuchtungs- und das Konvertermodul getrennt ausgetauscht werden können. Dies ist vorteilhaft, wenn die beiden Module sich unterschiedlich schnell verbrauchen, und daher in einer deutlich unterschiedlichen Anzahl von Messzyklen eingesetzt werden können.

In einer weiteren Ausführungsform der Erfindung enthalt das Disposable zusätzlich ein Partikelfilter, um eine Kontaminierung des Messgerätes mit Bakterien zu vermeiden. Geeignet sind z.B. entsprechende HEPA Filter (High Efficiency Particulate Airfilter). Der Filter sollte ausreichend feinporig sein, um Bakterien, oder Viren oder ähnliche Verunreinigungen aus dem Luftstrom zu filtern, gleichzeitig aber einen geringen Strömungswiderstand bieten.

Gemäß einer weiteren Ausführungsform wird das Konversionsmodul mit Luftentfeuchter im Sensorelement selbst integriert (hybrid oder monolithisch). Dies kann durch einen Mehrschichtaufbau erfolgen (z.B. Entfeuchterschicht, Oxidationskatalysatorschicht, Sensorschicht) oder durch einen monolithischen Aufbau (die Sensoroberfläche befindet sich auf dem gleichen Trägerkörper und ist homogen oder heterogen mit dem katalytisch aktiven Material und Entfeuchtungsmittel durchmischt).

Ferner kann ein Heizvorrichtung oder Trocknungsvorrichtung vorgesehen sein, um die Einrichtung zur Luftentfeuchtung zu regenerieren.

Bevorzugt kann eine Heizvorrichtung auf der Oberfläche oder im Material des Konversionsmoduls integriert vorliegen, welche die oxidative Fähigkeit und/oder die Kapazität zur Luftentfeuchtung des Moduls wieder regeneriert.

Die Heizvorrichtung kann automatisch in Betrieb gesetzt werden, gesteuert z.B. durch die Messung der Betriebsstunden oder durch die Messung des Gasflusses durch das Modul. In einer anderen Ausführungsform wird das Gasanalysegerät in wählbaren Zeitintervallen zur Qualitätskontrolle oder Kalibrierung mit einem Kalibriergas von definierter NO-Konzentration beaufschlagt. Dieser Kalibriervorgang kann auch dazu dienen, die Wirkungsrate des Konversionsmoduls zu messen und bei abfallender Wirkungsrate die Regenerierung zu aktivieren.

Das Trocknungsmittel ist gemäß einer Ausführungsform so ausgelegt (Materialüberschuß), dass auch bei schwankendem Feuchtigkeitsgehalt des Atems (z.B. aufgrund erhöhter Körpertemperatur) ein konstanter Feuchtigkeitsgehalt des Atems danach erzielt wird.

Es kann auch ein hypothetisch Feuchtigkeitsabhängiger Sensor eingesetzt werden.

Gemäß einer Ausführungsform ist das Trocknungsmittel auf einem offenporigen Matrixmaterial zur Einstellung eines definierten Atenwiderstandes aufgebracht ist. Alternativ kann auch an andere Stelle in der Vorrichtung ein offenporiges Material oder Fasergeflecht vorgesehen sein, um einen Atemwiderstand im Gerät zu definieren

Wesentliche Vorteile des Gesamtsystems liegen darin, dass eine nicht invasive Messmethode verwendet wird. Die Messungen sind in großer Anzahl wiederholbar und können somit auch zur Verlaufskontrolle bei Therapien, bei der Diagnose von Asthma bei Kindern, bei der Früherkennung von Asthma oder für vorbeugende medizinische Maßnahmen besonders eingesetzt werden. Durch Einsatz nicht verbrauchender Katalysatoren ist die Erfindungsgemäße Vorrichtung wartungsarm und ermöglicht kostengünstige Messungen. Das hier vorgestellte System ist deshalb auch für den Einsatz außerhalb von Kliniken und Arztpraxen geeignet.

## Patentansprüche

1. Vorrichtung (1) zur Messung mindestens eines Gasanalyten in Ausatemluft durch eine Gassensoreinheit (13) mit mindestens einem Gassensor (15), aufweisend eine Einrichtung zur Gaskonversion (17), wobei der Einrichtung zur Gaskonversion (17) eine Einrichtung zur Luftentfeuchtung (19) vorgeschaltet ist, wobei der mindestens eine Gasanalyt Stickstoffmonoxid ist und die Einrichtung zur Gaskonversion (17) eine Einrichtung (17) zur Oxidation von Stickstoffmonoxid zu Stickstoffdioxid ist.

2. Vorrichtung (1) nach Anspruch 1, wobei ein Indikator vorgesehen ist, der den Grad der Wasseraufnahme anzeigt oder anzeigt, wenn der Wassergehalt der Luft nach Durchströmen der Einrichtung zur Luftentfeuchtung (19) einen kritischen Schwellenwert übersteigt.

3. Vorrichtung (1) nach Anspruch 1 oder 2, ferner aufweisend ein chemisches Trocknungsmittel zur Luftentfeuchtung.

4. Vorrichtung (1) nach Anspruch 3, aufweisend eine Vorrichtung zur Regenerierung des chemischen Trocknungsmittels

5. Vorrichtung (1) nach Anspruch 1 oder 2, ferner aufweisend ein elektrisches Trocknungsmittel zur Luftentfeuchtung.

6. Vorrichtung (1) nach einem der vorangehenden Ansprüche, wobei die Einrichtung zur Luftentfeuchtung (13) und/oder die Einrichtung zur Gaskonversion (17) als Verbrauchsmittel in einer separaten Einheit (11) vorgesehen sind.

7. Vorrichtung (1) nach einem der vorangehenden Ansprüche, ferner aufweisend ein Partikelfilter.

8. Vorrichtung (1) nach einem der vorangehenden Ansprüche, ferner aufweisend ein Einwegventil, so dass ein Benutzer einmal in die Vorrichtung (1) ausgeatmete Luft nicht mehr ansaugen und wieder einatmen kann

9. Vorrichtung (1) nach Anspruch 1, wobei der Gassensor (15) ein NO₂-sensitiver FET-Sensor ist.

## Claims

1. Apparatus (1) for measuring at least one gas analyte in exhaled air using a gas sensor unit (13) with at least one gas sensor (15), having a device for gas conversion (17), wherein a device for air dehumidification (19) is arranged upstream of the device for gas conversion (17), wherein the at least one gas analyte is nitrogen monoxide and the device for gas conversion (17) is a device (17) for oxidizing nitrogen monoxide to nitrogen dioxide.

2. Apparatus (1) according to Claim 1, wherein provision is made for an indicator that displays the degree of water absorption or displays if the water content of the air exceeds a critical threshold after flowing through the device for air dehumidification (19).

3. Apparatus (1) according to Claim 1 or 2, further having a chemical desiccant for dehumidifying air.

4. Apparatus (1) according to Claim 3, having an apparatus for regenerating the chemical desiccant.

5. Apparatus (1) according to Claim 1 or 2, further having an electrical drying means for dehumidifying air.

6. Apparatus (1) according to one of the preceding claims, wherein the device for air dehumidification (13) and/or the device for gas conversion (17) are provided as a consumable in a separate unit (11).

7. Apparatus (1) according to one of the preceding claims, further having a particle filter.

8. Apparatus (1) according to one of the preceding claims, further having a one-way valve such that a user is no longer able to suck in and re-inhale air already exhaled into the apparatus (1).

9. Apparatus (1) according to Claim 1, wherein the gas sensor (15) is an NO2-sensitive FET-sensor.

## Revendications

1. Dispositif (1) pour mesurer au moins un analyte gazeux dans l'air d'expiration, au moyen d'une unité de détection de gaz (13) ayant au moins un capteur de gaz (15), présentant un dispositif de conversion de gaz (17), un dispositif de déshumidification de l'air (19) étant monté en amont du dispositif de conversion de gaz (17), l'au moins un analyte gazeux étant du monoxyde d'azote, et le dispositif de conversion de gaz (17) étant un dispositif (17) pour l'oxydation du monoxyde d'azote pour former du dioxyde d'azote.

2. Dispositif (1) selon la revendication 1, dans lequel un indicateur est prévu, lequel indique le degré d'absorption d'eau ou indique quand le taux d'humidité de l'air dépasse une valeur seuil critique après l'écoulement à travers le dispositif de déshumidification de l'air (19).

3. Dispositif (1) selon la revendication 1 ou 2, présentant en outre un agent dessiccatif chimique pour la déshumidification de l'air.

4. Dispositif (1) selon la revendication 3, présentant un dispositif pour régénérer l'agent dessiccatif chimique.

5. Dispositif (1) selon la revendication 1 ou 2, présentant en outre un agent dessiccatif électrique pour la déshumidification de l'air.

6. Dispositif (1) selon l'une quelconque des revendications précédentes, dans lequel le dispositif de déshumidification de l'air (13) et/ou le dispositif de conversion de gaz (17) sont prévus en tant que consommateurs dans une unité séparée (11).

7. Dispositif (1) selon l'une quelconque des revendications précédentes, présentant en outre un filtre à particules.

8. Dispositif (1) selon l'une quelconque des revendications précédentes, présentant en outre une soupape unidirectionnelle, de telle sorte qu'un utilisateur ne puisse plus aspirer et respirer à nouveau l'air expiré une fois dans le dispositif (1).

9. Dispositif (1) selon la revendication 1, dans lequel le capteur de gaz (15) est un capteur sensible aux NO₂.
